# EUROPEAN PATENT SPECIFICATION

(11) **EP 1 869 015 B1**
(45) Date of publication and mention of the grant of the patent: **12.01.2011**
(21) Application number: 06766260.1
(22) Date of filing: 16.03.2006
(51) Int. Cl.: C07D 401/12

(54) **AN IMPROVED PROCESS FOR THE MANUFACTURE OF RABEPRAZOLE SODIUM**
VERBESSERTES VERFAHREN ZUR ZUBEREITUNG VON RABEPRAZOL-NATRIUM
PROCEDE PERFECTIONNE DE FABRICATION DE RABEPRAZOLE SODIUM

(30) Priority: 30.03.2005 IN MU03702005
(43) Date of publication of application: 26.12.2007
(73) Proprietor: Lupin Limited, Mumbai 400 098, Maharashtra (IN)
(72) Inventor: SINGH, Girij, Pal, Pune 411 042, Maharashtra (IN); SIYAN, Rajinder, Singh, Pune 411 042 Maharashtra (IN); SINGH, Gurvinder, Pal, Pune 411 042 Maharashtra (IN); MAHALE, Rajendra Dagesing, Pune 411 042, Maharashtra (IN)
(74) Representative: Wibbelmann, Jobst
(86) International application number: PCT/IN2006/000104
(87) International publication number: WO 2006/120701

(56) References cited:
- WO-A-01/04109
- WO-A-03/082858
- US-A- 5 045 552

## Description

### Field of invention

The present invention relates to an improved process for the manufacture of Rabeprazole Sodium.

### Background of the invention

Rabeprazole Sodium (I) (CAS No. 117976-90-6) is chemically known as (±) sodium-2-[[[4-(3-methoxypropoxy)-3-methyl-pyridinyl]methyl]sulfinyl)-1H-benzimidazole.

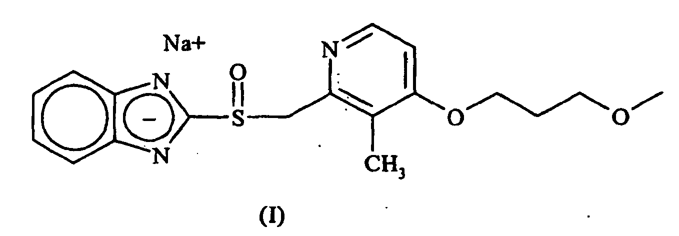

Rabeprazole sodium belongs to the class of H+ - K+ - ATPase inhibitors. Its intense effect of suppressing gastric acid secretion, and appropriate duration of action makes it extremely useful as an anti-ulcer agent.

Rabeprazole Sodium is commercially available in a pharmaceutical composition under the brand name ACIPHEX® marketed by Eisai and is covered under US 5045552 (JP priority application No. JP19870021989 19870202; JP19870077784 19870331; JP1986027053619861113)

US 5045552 discloses the preparation of Rabeprazole sodium by known traditional procedures, such as dissolution of the product in a mixture of stoichiometric quantity of aqueous sodium hydroxide and ethanol, then removal of water azeotropically, thereafter drying the residue at low pressure and then crystallization of the residue with less polar solvent such as diethyl ether, tert-butyl methyl ether.

This procedure of preparing sodium salt has numerous disadvantages such as large volume of solvents is required for azeotropic removal of water. The process also involves complete evaporation of the solvents and stirring the residue with solvents of low polarity like diethyl ether, tert-butyl methyl ether. During this operation the residue becomes very thick and hard which adheres to the walls of the reactor and stirrer blades, thus creating difficulties in agitation. Slow and interrupted agitation lead to formation of lumps, which yield courser product (*i.e*. with the particle size more than about 100 µm) with high solvent entrapment, necessiting longer drying time. Not only it increases steam cost but also increases the batch cycle time because of higher occupancy in the unit operation of drying, thus rendering the process economically not very viable.

WO 03/101452 discloses a method for the preparation of Rabeprazole sodium comprising dissolving Rabeprazole base in aqueous sodium hydroxide and then subjecting to lyophilization. The process suffers from two major disadvantages *viz*.1) Employment of lyophilization operation, which tends to increase the cost of production. 2) lyophilization involves large capital expenditure.

It would be evident from the literature cited hereinbefore that the isolation of Rabeprazole sodium suffers from various disadvantages. Therefore, a need exists for developing an economically and technologically superior process, which would
(a) carry out the preparation of sodium salt with comparatively smaller volume of solvent;
(b) be easy to operate in commercial scales;
(c) achieve a optimum particle size with minimum solvent usage thereby reducing the drying time;
(d) not employ substantial capital expenditure item like a lyophilizer.

The inventors have now found a process for the isolation of Rabeprazole sodium by judicial use of solvents, which satisfies the above criteria.

### Objects of the invention

Thus an object of the present invention is to provide an improved industrial process for the preparation of amorphous Rabeprazole sodium with mean particle diameter of below 50 µm to achieve better bioavailability.

Another object of the present invention is to provide a process for the isolation of amorphous Rabeprazole sodium, which uses comparatively lower volume of solvent.

A further object of the present invention is to provide a process for the isolation of Rabeprazole sodium in amorphous form, which is easy to operate in commercial scale.

Another object of the present invention is to provide an improved industrial process for the preparation of amorphous Rabeprazole sodium with better material usage efficiency *i.e.* improved yields than reported in the literature.

A further object of the present invention is to provide an improved industrial process for the preparation of amorphous Rabeprazole sodium with pharmaceutically acceptable purity.

### Summary of invention

Thus in the present invention there is provided a process for manufacture of amorphous Rabeprazole sodium with mean particle diameter between 10 to 55 µm said process comprising
a) addition of Rabeprazole to an aqueous sodium hydroxide;
b) addition of ethyl alcohol to the solution obtained in step 'a';
c) distillation of solvents from the solution obtained in step 'b' till thick mass is obtained;
d) addition of an organic solvent selected from C3 to C8 straight chain or branched aliphatic ester, chlorinated aliphatic hydrocarbon, cyclic ethers and mixtures thereof to the residue to obtain a clear solution;
e) addition of this clear solution to an anti-solvent under agitation and
f) isolation of the product.

### Detailed description of the invention:

The solution of Rabeprazole in sodium hydroxide is treated with activated charcoal and then filtered. Ethyl alcohol is added to this solution and distilled. Distillation is continued till a thick mass is obtained which is then dissolved in organic solvent.

The organic solvent used is selected from ethyl acetate, dichloromethane, chloroform, butyl acetate, ethanol, isopropyl alcohol, methanol, tetrahydrofuran and mixtures thereof. The solvent is used in an amount of 2 to 5 ml per gram of rabeprazole.

The anti-solvent used is selected from various organic solvents in which Rabeprazole sodium is sparingly soluble. The said anti-solvent includes diisopropyl ether, diethyl ether, methyl tert-butyl ether and mixtures thereof. The anti- solvent is used in an amount of 10 to 15 ml per gram of rabeprazole.

Finally amorphous rabeprazole sodium is isolated by filtration. Filtration includes use of all conventional filters such as vacuum filter, pressure filter or centrifugation.

Isolation of amorphous solid many a time leads to agglomeration and resulting in a sticky mass adhering strongly to the reaction vessel, agitator etc. if solvent with proper polarity is not used for isolation. Such a mass thus formed disintegrate into lumps and give rise to bigger particles with high solvent entrapment. Bigger particles have lower surface area, hence requires prolonged time for drying. The amorphous rabeprazole sodium thus formed is of smaller particle size (mean particle diameter 10 to 55 µm) and is thus capable of faster drying and does not require use of lyophilizer. The yield of the product obtained by the present process is 80 to 90 % calculated w.r.t. Rabeprazole and it has a purity level not less than 99 %. The so obtained product is found to be stable under 75% relative humidity at a temperature of 40°C. The product has better bioavailability than that of the product with particle size of above 100 µm.

The present invention is illustrated in more detail by referring to the following examples, which are not to be construed as limiting the scope of the invention.

### Example 1:

### Isolation of Rabeprazole sodium using ethyl acetate as solvent:

Rabeprazole (50 gm) was dissolved in solution of sodium hydroxide (5.39 gm ) in demineralized water (162.5 ml). The solution was extracted with dichloromethane (100 ml) twice. The aqueous layer was then treated with neutral activated charcoal (1 gm) for 30 minutes at 28 to 35°C. Carbon was removed by filtration and the residue washed with demineralized water (12.5 ml). Ethyl alcohol (50 ml) was added to the clear filtrate. The solution was concentrated to thick mass under vacuum at 40 - 45°C. The thick mass was dissolved in ethyl alcohol (100 ml) and was again concentrated to a thick mass under vacuum at 40 - 45°C. The residue was then dissolved in ethyl acetate (100 ml) and the solution was concentrated to a thick oily mass under vacuum. The residue thus obtained was dissolved in ethyl acetate (100 ml) and the solution was added slowly over a period of 20 to 30 minutes to diisopropyl ether (500 ml). The slurry thus obtained was stirred for 60 minutes at 28 to 35°.. The solid was filtered and washed with diisopropyl ether. Then the solid was dried at 45 - 50° to get Rabeprazole sodium as a white amorphous solid (powder X-ray diffraction of the product does not show any sharp peak, shows only the base line), with mean particle diameter ranging between 10 to 50µm. Yield: 45 gm (85 %), Assay: 99.5 % (by HPLC). IR Spectra (KBr, cm⁻¹): 3382, 2927, 1583, 1462, 1384, 1298, 1269, 1190, 1157, 1093, 1018, 745. H NMR Spectra [200 M Hz, CD3OD] δ (ppm): 8.23 - 8.25 (1H, d, ArH); 7.57 - 7.62 (2H, m, ArH); 7.0 - 7.09 (2H, m, ArH); 6.87 - 6.90 (1H, d, ArH); 4.57 - 4.63 (2H, d, O=S-CH2-Ar); 4.0 - 4.1 (2H, t, -O-CH2-CH2-); 3.49 - 3.55 (2H, t, -CH2-O-CH3); 3.31 (3H, s, -OCH3); 2.1 (3H, s, Ar-CH3); 1.96 - 2.0 (2H, t, -CH2-CH2-CH2-).

### Example 2:

### Isolation of Rabeprazole sodium using dichloromethane as solvent:

Rabeprazole (50 gm) was dissolved in solution of sodium hydroxide (5.4 gm) in demineralized water (150 ml). The solution was extracted with dichloromethane (100 ml) twice. The aqueous layer was then treated with neutral activated charcoal (1 gm) for 30 minutes at 28 to 35°C. Carbon was removed by filtration and the residue washed with demineralized water (12.5 ml). Ethyl alcohol (50 ml) was added to the clear filtrate. The solution was concentrated to thick mass under vacuum at 40 - 45°C. The thick mass was dissolved in ethyl alcohol (100 ml) and was again concentrated to a thick mass under vacuum at 40 - 45°C. The residue was then dissolved in dichloromethane (150 ml) and the solution was filtered through 0.5-micron filter pad. The clear solution thus obtained was added slowly over a period of 20 to 30 minutes to diisopropyl ether (500 ml). The slurry thus obtained was stirred for 30 minutes at 28 to 35°. The solid was filtered and washed with diisopropyl ether. Then the solid was dried at 45 - 50° to get Rabeprazole sodium as a white amorphous solid (as evident from powder X-ray diffraction of the product), with mean particle diameter ranging between 15 to 55µm. Yield: 44 gm (83 %), Assay: 99.5 % (by HPLC)

### Reference Example 3:

Preparation of sodium salt of Rabeprazole is carried out as per the procedure described in example 6 of US 5045552 and the particle size and drying time for the finished product were compared with that of the product obtained from examples 1 and 2 **(Table 1).**

**Table. 1**

| **Sr. No.** | **Process utilized** | **Particle size*** | **Drying time** |
|---|---|---|---|
| **1.** | Example 6 of US 5045552 | 80 to 150 µm | 51 hrs. |
| **2.** | Example 1 of the present application | 10 to 50 µm | 20 hrs. |
| **3.** | Example 2 of the present application | 15 to 55 µm | 20 hrs. |

| | | | |
|---|---|---|---|
| ** Particle size measured on MALVERN*® *mastersizer 2000 (Dispersant used: light liquid paraffin).* | | | |

It is evident from the data as shown in Table 1 that the process of Examples 1 and 2, which utilizes the requisite combination of, defined solvents and anti solvents is able to avoid formation of lumps and hence it leads to smaller particles which makes the operation of drying easy and less time consuming. Further the process of the present invention uses lesser amount of solvents and yet achieves the desired result.

## Claims

1. A process for manufacture of amorphous Rabeprazole sodium with mean particle diameter between 10 to 55 µm said process comprising
a) addition of Rabeprazole to an aqueous sodium hydroxide;
b) addition of ethyl alcohol to the solution obtained in step 'a';
c) distillation of solvents from the solution obtained in step 'b' till thick mass is obtained;
d) addition of an organic solvent selected from C3 to C8 straight chain or branched aliphatic ester, chlorinated aliphatic hydrocarbon, cyclic ethers and mixtures thereof to the residue to obtain a clear solution;
e) addition of this clear solution to an anti-solvent under agitation and
f) isolation of the product.

2. The process as claimed in claim 1 wherein the aqueous solution of Rabeprazole sodium is treated with charcoal.

3. The process as claimed in claim 1 wherein the organic solvent used is selected from ethyl acetate, dichloromethane, chloroform, butyl acetate, tetrahydrofuran and mixtures thereof.

4. The process as claimed in claim 1 wherein the anti-solvent used is selected from the group of C4 to C10 straight chain or branched aliphatic ethers and mixtures thereof.

5. The process as claimed in claim 4 wherein the anti-solvent used is selected from diisopropyl ether, diethyl ether, methyl tert-butyl ether and mixtures thereof.

6. A process as claimed in any preceding claim wherein the isolation of the product is carried out by filtration.

## Patentansprüche

1. Verfahren zur Herstellung von amorphem Rabeprazol-Natrium mit einem mittleren Teilchendurchmesser zwischen 10 und 55 µm, wobei das Verfahren umfasst
a) Zugeben von Rabeprazol zu wässrigem Natriumhydroxid;
b) Zugeben von Ethylalkohol zu der in Schritt 'a' erhaltenen Lösung;
c) Destillieren der Lösungsmittel aus der in Schritt 'b' erhaltenen Lösung bis eine dickliche Masse erhalten wird;
d) Zugeben von einem organischen Lösungsmittel ausgewählt aus C3- bis C8-geradkettigen oder verzweigten aliphatischen Estern, chlorierten aliphatischen Kohlenwasserstoffen, zyklischen Ethern oder Mischungen davon zu dem Rückstand, um eine klare Lösung zu erhalten;
e) Zugeben dieser klaren Lösung zu einem Antilösungsmittel unter Rühren und
f) Isolieren des Produkts.

2. Verfahren wie beansprucht in Anspruch 1, wobei die wässrige Rabeprazol-Natriumlösung mit Aktivkohle behandelt wird.

3. Verfahren wie beansprucht in Anspruch 1, wobei das verwendete organische Lösungsmittel ausgewählt ist aus Ethylacetat, Dichlormethan, Chloroform, Butylacetat, Tetrahydrofuran und Mischungen davon.

4. Verfahren wie beansprucht in Anspruch 1, wobei das verwendete Antilösungsmittel ausgewählt ist aus der Gruppe aus C4- bis C10-geradkettigen oder verzweigten aliphatischen Ethern und Mischungen davon.

5. Verfahren wie beansprucht in Anspruch 4, wobei das verwendete Antilösungsmittel ausgewählt ist aus Diisopropylether, Diethylether, Methyl-tert-butylether und Mischungen davon.

6. Verfahren wie beansprucht in einem der vorangegangenen Ansprüche, wobei die Isolierung des Produkts mittels Filtrieren ausgeführt wird.

## Revendications

1. Procédé de fabrication de Rabéprazole sodique amorphe avec un diamètre moyen de particules entre 10 à 50 µm, ledit procédé comprenant
a) l'addition de Rabéprazole à un hydroxyde de sodium aqueux ;
b) l'addition d'alcool éthylique à la solution obtenue à l'étape « a » ;
c) la distillation des solvants de la solution obtenue à l'étape « b » jusqu'à ce qu'une masse épaisse soit obtenue ;
d) l'addition d'un solvant organique choisi parmi un ester aliphatique à chaîne droite ou ramifié en C3-C8, un hydrocarbure aliphatique chloré, des éthers cycliques et des mélanges de ceux-ci au résidu pour obtenir une solution transparente ;
e) l'addition de cette solution transparente à un anti-solvant sous agitation et
f) l'isolation du produit.

2. Procédé selon la revendication 1, dans lequel la solution aqueuse de Rabéprazole sodique est traitée avec du charbon de bois.

3. Procédé selon la revendication 1, dans lequel le solvant organique utilisé est choisi parmi l'acétate d'éthyle, le dichlorométhane, le chloroforme, l'acétate de butyle, le tétrahydrofurane et des mélanges de ceux-ci.

4. Procédé selon la revendication 1, dans lequel l'anti-solvant utilisé est choisi parmi le groupe des éthers aliphatiques à chaîne droite ou ramifiés en C4-C10 et des mélanges de ceux-ci.

5. Procédé selon la revendication 4, dans lequel l'anti-solvant utilisé est choisi parmi l'éther diisopropylique, l'éther diéthylique, l'éther méthyl-tert-butylique et des mélanges de ceux-ci.

6. Procédé selon une quelconque revendication précédente, dans lequel l'isolation du produit est effectuée par filtration.
